(19) Europäisches Patentamt
European Patent Office
Office européen des brevets

(11) Publication number: **0 234 480 A2**

(12) **EUROPEAN PATENT APPLICATION**

(21) Application number: 87102210.9

(22) Date of filing: 17.02.87

(51) Int.Cl.³: C 07 D 339/00
C 07 D 409/06, A 61 K 31/38-5

(30) Priority: 21.02.86 JP 36798/86
22.02.86 JP 37838/86

(43) Date of publication of application:
02.09.87 Bulletin 87/36

(84) Designated Contracting States:
BE CH DE FR GB IT LI LU NL SE

(71) Applicant: NIHON NOHYAKU CO., LTD.
1-2-5, Nihonbashi
Chuo-ku Tokyo(JP)

(72) Inventor: Kubota, Shuhei
J-200, Beverly Boulevard
Upper Darby Pennsylvania, 19082(US)

(72) Inventor: Nagamine, Masashi
Nihon Nohyaku Maruhashiro 4-6, Maruhashicho
Nishinomiya-shi(JP)

(72) Inventor: Taninaka, Kuniaki
Uchiage Danchi A 1-503 386-6 Oaza Uchiage
Neyagawa-shi(JP)

(72) Inventor: Nakayama, Keisuke
Nihon Nohyaku Ichikawaryo 13-21, Owada-2-chome
Ichikawa-shi(JP)

(72) Inventor: Konaka, Shigeo
9-4-213, Takawashi-4-chome
Habikino-shi(JP)

(72) Inventor: Uchida, Matazaemon
14-22, Daishicho
Kawachinagano-shi(JP)

(74) Representative: Patentanwälte Grünecker, Kinkeldey, Stockmair & Partner
Maximilianstrasse 58
D-8000 München 22(DE)

(54) **1,3-diketone derivatives and their use.**

(57) A 1,3-diketone derivative represented by the general formula (I)

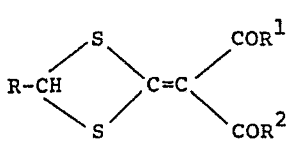

(I)

wherein $R^1$ denotes $C_1$-$C_5$ alkyl group; a phenyl group or a phenyl group substituted with halogen atom, $C_1$-$C_5$ alkyl group, $C_1$-$C_5$ alkoxy group; $R^2$ denotes a phenyl group; a phenyl group substituted with 1 to 3 groups selected from the group consisting of a halogen atom, $C_1$-$C_5$ alkyl group, an $C_1$-$C_5$ alkoxy group, a phenyl group, a phenoxy group, and a benzyloxy group optionally having a halogen atom as a substituent on the phenyl ring; naphthyl group; a furyl group; a thienyl group; or a pyridyl group; provided that when $R^2$ is a phenyl group $R^1$ is a group other than a methyl or phenyl group; and R denotes a hydrogen atom, a methyl group, and a phenyl group and a pharmaceutical composition containing the same as active ingredient.

EP 0 234 480 A2

Croydon Printing Company Ltd.

# 1,3-DIKETONE DERIVATIVES AND THEIR USE

This invention relates to a 1,3-diketone derivative represented by the general formula (I)

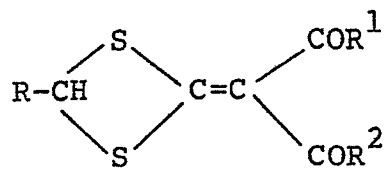

(I)

wherein $R^1$ denotes $C_1$-$C_5$ alkyl group; a phenyl group; or a phenyl group substituted with halogen atom, $C_1$-$C_5$ alkyl group, $C_1$-$C_5$ alkoxy group; $R^2$ denotes $C_1$-$C_5$ alkyl group; a phenyl group; a phenyl group substituted with 1 to 3 groups selected from the group consisting of a halogen atom, $C_1$-$C_5$ alkyl group, $C_1$-$C_5$ alkoxy group, a phenyl group, a phenoxy group, and a benzyloxy group optionally having a halogen atom as a substituent on the phenyl ring; naphthyl group; a furyl group, a thienyl group, or a pyridyl group; and R denotes a hydrogen atom, a methyl group or a phenyl group, a pharmaceutical composition for treating hepatic disorders containing the said derivative as an active ingredient.

The compound represented by the general formula (I) has, for example, a liver function activating effect, and hence is useful as an active ingredient for pharmaceutical composition for treating hepatic disorders in men and animals.

Some compounds represented by the general formula (I) in chemical structure are already known in Litrature. [Tetrahedron, 30, 93 - 104 (1974).]

However, their biological activity has never been reported.

The known compound is shown below:

1-benzoyl-1-(1,3-dithietan-2-ylidene) acetone.

1-benzoyl-1-(1,3-dithietan-2-ylidene) acetone.

1-acetyl-1-(1,3-dithietan-2-ylidene) acetone.

In the general formula (I), the alkyl group denoted by $R_1$ and $R_2$, and the alkyl moiety of the alkoxy group denoted by $R^2$ include, for example, a lower alkyl group such as methyl, ethyl, n-propyl, iso-propyl, n-butyl, iso-butyl, sec-butyl, tert-butyl, n-amyl, iso-amyl, and sec-amyl.

The compound represented by the general formula (I) can be synthesized according to the reaction route shown schematically below.

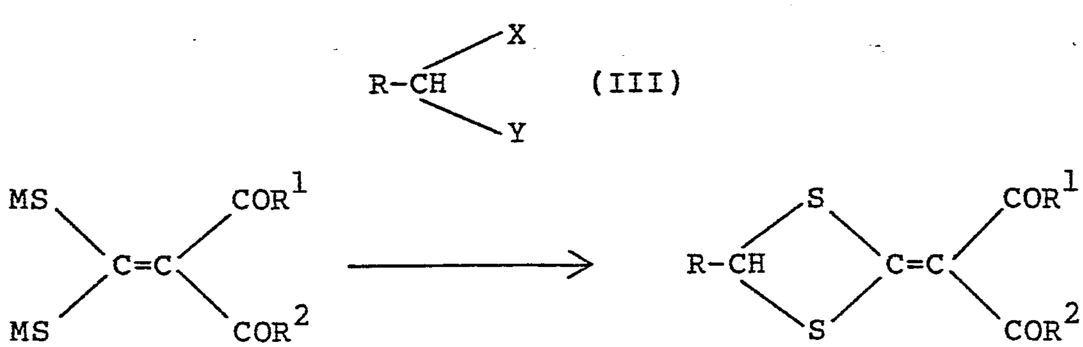

(III)

(II)

(I)

In the above formulas, $R^1$ denotes $C_1$-$C_5$ alkyl group; a phenyl group or a phenyl group substituted with halogen atom, $C_1$-$C_5$ alkyl group, $C_1$-$C_5$ alkoxy group; $R^2$ denotes $C_1$-$C_5$ alkyl group; a phenyl group; a phenyl group substituted with 1 to 3 groups selected from the group consisting of a halogen atom, an alkyl group, an alkoxy group, a phenyl group, a phenoxy group, and a benzyloxy group optionally having a halogen atom as a substituent on the phenyl ring; naphthyl group: a furyl group, a thienyl group, or a pyridyl group; R denotes a hydrogen atom, a methyl group, or a phenyl group; M denotes an alkali metal atom; and X and Y denotes a halogen atom.

Thus, the 1,3-diketone derivative represented by the general formula (I) can be obtained by reacting a compound represented by the general formula (II) with a compound represented by the formula (III) in an inert solvent.

The solvent which can be used herein are preferably a dipolar aprotic solvent such as dimethyl sulfoxide, acetonitrile, diethylformamide, N,N-diethylacetamide, N,N'-dimethylethyleneurea, dimethylformamide, hexamethylphosphoramide, N-methylpyrrolidone, and N,N-diethylacetamide, acetone, methylethylketone.

The reaction temperature can be selected as desired out of the range of room temperature to the boiling range of the solvent used.

The compound represented by the general formula

(II), used as the starting material, can be synthesized through the reaction route shown below.

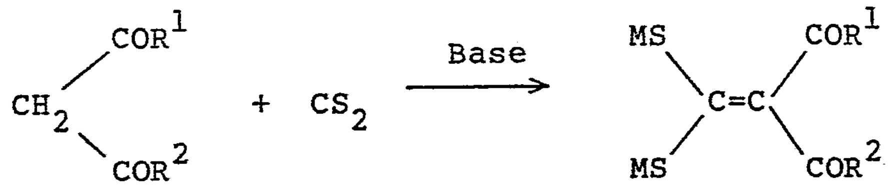


(V) (II)

In the above formulas, $R^1$, $R^2$ and M have the same meanings as mentioned above.

Thus, the compound can be obtained by reacting a compound represented by the general formula (V) with carbon disulfide in the presence of a base in an inert solvent.

Examples of the base which can be used herein include potassium carbonate, sodium hydroxide, and potassium hydroxide, sodium ethylate, potassium t-butoxide.

The inert solvent to be used is preferably a dipolar aprotic solvent such as dimethyl sulfoxide, dimethylformamide, hexaethylenephosphoramide, N-methylpyrrolidone, and N,N-diethylacetamide.

The reaction temperature is preferably in the range of 15°C to 30°C.

The compound represented by the general formula (II) may be used in the subsequent reaction either after isolated or without isolated.

Typical examples of the compound represented by the general formula (I) are shown in Table 1.

Among those compounds exemplified in Table 1 below is preferred a compound whose R is a hydrogen atom. Especially preferred one is a compound represented by the general formula (Ia):

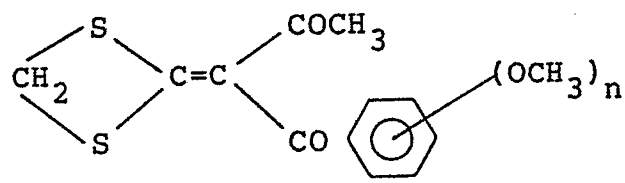

(Ia)

wherein n is an integer of 1 or 2.

Table 1

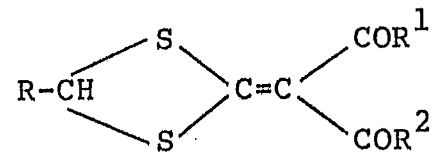

| Compound No. | R | $R^1$ | $R^2$ | Physical property: m.p. (°C) or refractive index |
|---|---|---|---|---|
| 1 | H | $CH_3$ | 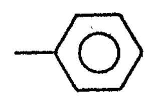 | m.p. 99.0 |
| 2 | H | $CH_3$ | 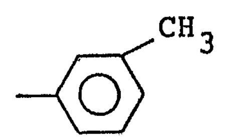 | m.p. 108.0 |
| 3 | H | $CH_3$ | 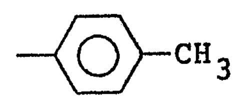 | m.p. 151.5 |
| 4 | H | $CH_3$ | 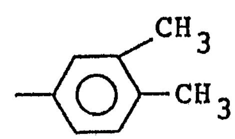 | m.p. 128.0 |
| 5 | H | $CH_3$ | 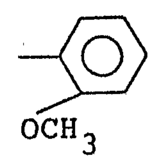 | m.p. 102.0 |

Table 1 (cont'd)

| Compound No. | R | $R^1$ | $R^2$ | Physical property: m.p. (°C) or refractive index |
|---|---|---|---|---|
| 6 | H | $CH_3$ | 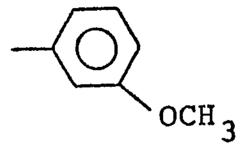 | m.p. 97.0 |
| 7 | H | $CH_3$ | 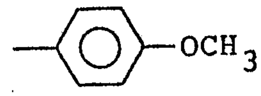 | m.p. 135.0 |
| 8 | H | $CH_3$ | 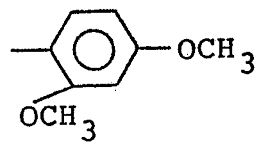 | m.p.126.0-127.0 |
| 9 | H | $CH_3$ | 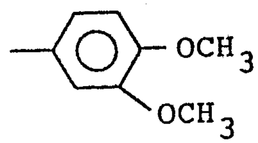 | m.p. 165.0 |
| 10 | H | $CH_3$ | 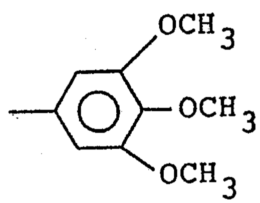 | m.p. 174.0-176.0 |

Table 1 (cont'd)

| Compound No. | R | $R^1$ | $R^2$ | Physical property: m.p. (°C) or refractive index |
|---|---|---|---|---|
| 11 | H | $CH_3$ | 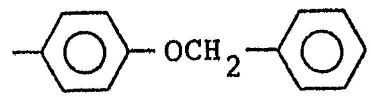 | m.p. 170.0 |
| 12 | H | $CH_3$ | 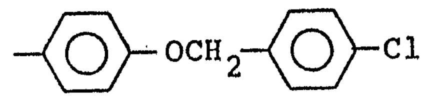 | m.p. 179.0-180.0 |
| 13 | H | $CH_3$ | 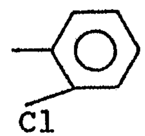 | m.p. 100.0 |
| 14 | H | $CH_3$ | 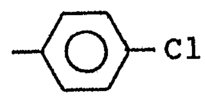 | m.p. 135.0 |
| 15 | H | $CH_3$ | 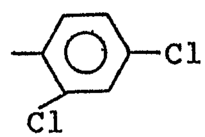 | m.p. 124.5-125.5 |
| 16 | H | $CH_3$ | 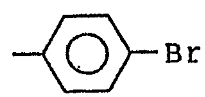 | m.p. 134.0-136.0 |

Table 1 (cont'd)

| Compound No. | R | $R^1$ | $R^2$ | Physical property: m.p. (°C) or refractive index |
|---|---|---|---|---|
| 17 | H | $CH_3$ | 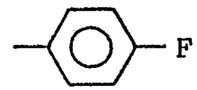 | m.p. 131.5 |
| 18 | H | $CH_3$ | $CH_3$ | m.p. 115.0 |
| 19 | H | $CH_3$ | 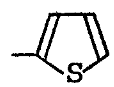 | m.p. 120.0-121.0 |
| 20 | H | $CH_3$ | 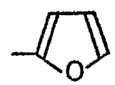 | m.p. 121.0 |
| 21 | H | $CH_3$ | 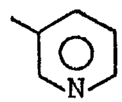 | m.p. 109.5-110.0 |
| 22 | H | 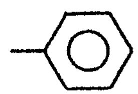 | 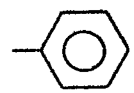 | m.p. 149.0-150.0 |
| 23 | 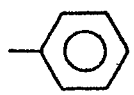 | $CH_3$ | 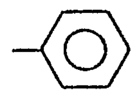 | paste |

Table 1 (cont'd)

| Compound No. | R | $R^1$ | $R^2$ | Physical property: m.p. (°C) or refractive index |
|---|---|---|---|---|
| 24 | 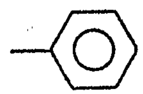 | $CH_3$ | 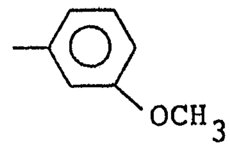 | m.p. 90.5 |
| 25 | $CH_3$ | $CH_3$ | $CH_3$ | m.p. 101.0-102.0 |
| 26 | H | $CH_3$ | 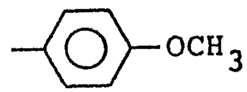 | paste |
| 27 | H | $C_2H_5$ | 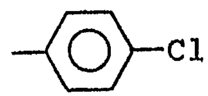 | paste |
| 28 | H | $C_3H_{7-i}$ | 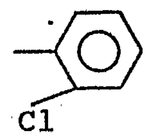 | m.p. 104-105 |
| 29 | H | $CH_3$ | 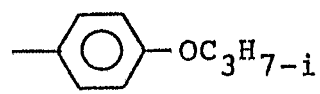 | m.p. 147-150 |

Table 1 (cont'd)

| Compound No. | R | $R^1$ | $R^2$ | Physical property: m.p. (°C) or refractive index |
|---|---|---|---|---|
| 30 | H | 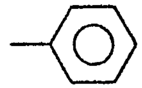 | 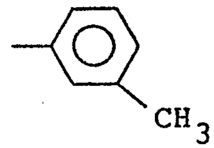 | m.p. 165-166 |
| 31 | H | 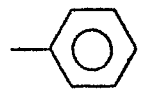 | 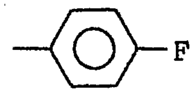 | m.p. 167-168 |
| 32 | H | $CH_3$ | 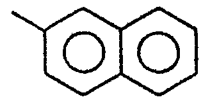 | paste |
| 33 | H | $C_2H_5$ | 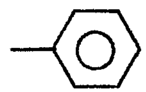 | m.p. 141.7 |

Note: 1) NMR data of compound No. 23

$\delta_{CDCl_3}^{TMS}$ ppm: 2.33 (3H, s), 7.10-7.95 (11H, m)

Note: 2) NMR data of compound no. 26

$\delta_{CDCl_3}^{TMS}$ ppm: 1.95 (3H, d), 2.00 (3H, s), 3.83 (3H, s), 3.90 (3H, s), 6.43-7.50 (3H, m)

Table 1 (cont'd)

Note: 3) NMR data of compound No. 27

$\delta^{TMS}_{CDCl_3}$ ppm: 0.96 (3H, t), 2.30 (2H, q), 3.77 (3H, s), 3.87 (3H, s), 4.10 (2H, s), 6.33-7.30 (3H, m)

Note: 4) NMR data of compound No. 32

$\delta^{TMS}_{CDCl_3}$ ppm: 1.67 (3H, s), 4.10 (2H, s) 7.34-8.05 (7H, m)

The 1,3-diketone derivative represented by the general formula (I) caused no toxic symptom nor death in mice or rats even after administered continually for two weeks at a dose of 300 mg/kg/day to the mice or rats, which reveals the markedly low toxicity of the compound of this invention. For example, $LD_{50}$ value (acute oral toxicity to male rat) of the compound No. 8 is more than 1,000 mg/kg.

The compound represented by the general formula (I) is useful as a medicinal agent for treating liver diseases. For example, while it is known that hepatic disorders can be experimentally produced in healthy test animals by administering various agents such as carbon tetrachloride to the animals, as disclosed for example in U.S. Patent No. 4,118,506, it has been found that the compound represented by the general formula (I) gives a marked effect of suppressing the lowering of liver functions or improving said functions when administered orally or parenterally (for example by injection) to test animals which have hepatic disorders of various pathologic models experimentally produced therein. Accordingly, the compound represented by the general formula (I) is useful as a medicinal agent for curing or preventing hepatic disorders in men and animals. Thus, it can be used as a curative for acute or chronic hepatic disorders of men and animals produced by various causes, for example jecur adiposum, alcoholic hepatitis, hepatitis, toxic liver disorders, cardiac cirrhosis, cholestatic liver disorder,

or hepatocirrhosis which is the final state of these diseases.

Accordingly, the term "a pharmaceutical composition for treating hepatic disorders" as used in this invention means a medicinal agent for curing and/or preventing various disorders in liver by utilizing the pharmacological actions manifested in liver as mentioned above including the action of activating liver functions and the action of preventing and curing hepatic disorders.

The compound represented by the general formula (I) can be used as a medicinal agent for treating hepatic disorders in the form as it is; it may also be formulated, according to conventional pharmaceutical procedures, as a mixture thereof with a pharmaceutically acceptable diluents and/or other pharmacologically active substances. Further, it may be formulated into a dose unit form. Examples of the form which the compound can take as a medicinal agent include: powders, granules, tablets, dragée, capsules, pills, suspensions, solutions, liquid, emulsions, ampules, injections, and isotonic solutions.

The modes of preparing the compound of this invention into a phamaceutical composition include one wherein the compound represented by the general formula (I) is contained as a mixture thereof with one or more pharmaceutically acceptable diluents.

The "diluent" referred to herein means a material other than the compound represented by the general formula (I). It may be in the form of solid,

semisolid, liquid, or ingestible capsules. Examples of the diluents include excipients, fillers, binders, moistening agents, disintegrators, surfactants, lubricants, dispersants, buffering agents, flavoring agents, odor correctives, coloring agents, flavors, preservatives, solubilizing aids, solvents, coating agents, and sugar-coating agents. However, they are not limited to these. Further, they may be used as a mixture of one or more kinds thereof. Sometimes, these pharmaceutically acceptable diluents are used as a mixture thereof with other pharmacologically active substances.

The pharmaceutical composition according to this invention may be prepared by any method known in the art. For instance, the active ingredient is mixed with a diluent and made up, for example, into granules. The resulting composition is then formed, for example, into tablets. Preparations to be administered parenterally should be made aseptic. Further, as occasion demands, they should be made isotonic with blood.

In this invention, since the compound represented by the general formula (I) mentioned above can be itself make a medicinal agent for treating liver diseases, the active ingredient is generally contained in the composition in a proportion of 0.01 to 100% by weight.

When the compound is made into a preparation in the form of dose unit, the individual parts of the preparation which form said preparation may be either in the same shape or in shapes different from each other. For

example, the following shapes are often adopted: tablets, granules, pills, powders, dragée, capsules, ampules, and the like.

The medicinal agent for treating hepatic disorders according to this invention can be applied to men and animals for the purpose of preventing and treating hepatic disorders therein, in a manner conventional in the art. It is administered orally or parenterally. Oral administration referred to herein includes sublingual administration. Parenteral administration includes herein administrations conducted by means of injections (including, for example, subcutaneous, intramuscular or intravenous injection and instillation).

The dose of the medicinal agent of this invention varies depending upon various factors including whether it is applied to animals or men, difference in susceptibility, age, sex, body weight, the method, time, and interval of administration, the condition of diseases, physical condition, the properties of the pharmaceutical composition, the kind of the preparation, and the kind of the active ingredient.

Accordingly, sometimes those doses may be sufficient which are lower than the minimum of the dose range shown below, whereas sometimes it becomes necessary to administer an amount exceeding the upper limit of the dose shown below.

When the pharmaceutical composition is to be administered in a large amount, it is preferably administered divided in several doses per day.

In order to obtain effective results in application to animals, the agent is advantageously administered at a dose, in terms of the active ingredient, in the range of 0.1 to 500 mg, preferably 0.1 to 30 mg, per 1 kg of body weight per day in oral administration, and 0.01 to 250 mg, preferably 0.1 to 25 mg, per 1 kg of body weight per day in the case of parenteral administration.

The doses necessary for obtaining effective results in application to men are, judged from the effective doses in animals and in consideration of difference in susceptibility and safety, advantageously selected, for example, from the following dose range. In oral administration the dose is 0.1 to 200 mg, preferably 0.5 to 50 mg, per kg of body weight per day, and in parenteral administration it is 0.01 to 100 mg, preferably 0.1 to 25 mg, per kg of body weight per day.

Example

This invention will be described in detail below with reference to Examples, but it is in no way limited thereto.

First, synthesis examples of this invention are shown below.

Synthesis Example 1

Synthesis of 1-(4-bromobenzoyl)-1-(1,3-dithietan-2-ylidene)acetone (compound No. 16)

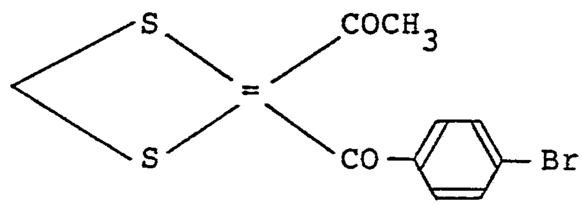

To a mixture of 2.43 g of p-bromobenzoylacetone, 0.8 g of carbon disulfide, and 10 ml of dimethyl sulfoxide, well stirred at room temperature, was added 1.5 g of anhydrous potassium carbonate. After being stirred as it was for further one hour, the reaction liquid was cooled with ice water, and 1.74 g of dibromomethane was added thereto to effect reaction. After 2 hours of stirring at room temperature, water was added to the reaction mixture, and the resulting mixture was extracted with ethyl acetate. The ethyl acetate layer obtained was washed with water, dried over anhydrous sodium sulfate, and stripped of the solvent by evaporation to give crude crystals. The crystals were recrystallized from a solvent mixture consisting of isopropanol and n-hexane. Yield, 20 g (60.8% yield); m.p., 134° - 136°C.

Synthesis Example 2

Synthesis of 1-(1,3-dithietan-2-ylidene)-1-(2-methoxybenzoyl)acetone (compound No. 5)

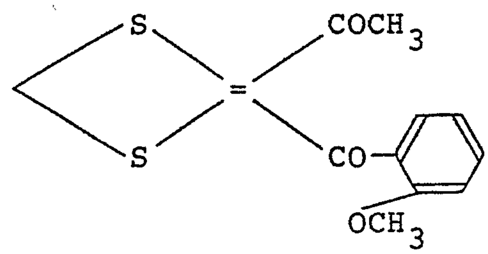

To a mixture of o-methoxybenzoylacetone, 0.8 g of carbon disulfide, and 10 ml of dimethyl sulfoxide, well stirred at room temperature, was added 1.5 g of anhydrous potassium carbonate. After being stirred as it was for further one hour, 0.85 g of dichloromethane was added thereto to effect reaction. After 1 hours of stirring at room temperature, water was added to the reaction mixture, and the resulting mixture was extracted with ethyl acetate. The ethyl acetate layer obtained was washed with water, dried over anhydrous sodium sulfate, and stripped of the solvent by evaporation to give crude crystals. The crystals were recrystallized from a solvent mixture consisting of isopropanol and n-hexane. Yield, 0.98 g (34.6% yield); m.p., 102°C.

Synthesis Example 3

Synthesis of 1-(3,4-dimethylbenzoyl)-1-(1,3-dithietan-2-ylidene)acetone (compound No. 4)

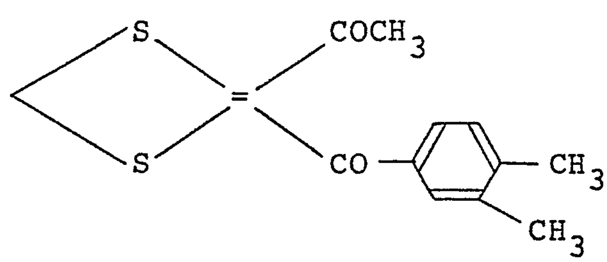

To a mixture of 1.93 g of 3,4-dimethylbenzoylacetone, 0.8 g of carbon disulfide, and 10 ml of dimethylformamide, well stirred at room temperature, was added 1.5 g of anhydrous potassium carbonate. After being stirred as it was for further 2 hours, the reaction liquid was cooled with ice water, and 1.74 g of dibromomethane was added thereto to effect reaction. After 2 hours of stirring at room temperature, water was added to the reaction mixture, and the resulting mixture was extracted three times with ethyl acetate. The ethyl acetate layer obtained was washed with water, dried over anhydrous sodium sulfate, and stripped of the solvent by evaporation to give crude crystals. The crystals were recrystallized from a solvent mixture consisting of isopropanol and n-hexane. Yield, 1.58 g (56.2% yield); m.p., 128°C.

Synthesis Example 4

Synthesis of 1-(1,3-dithietan-2-ylidene)-1-(2-thiophenecarbonyl)acetone (compound No. 19)

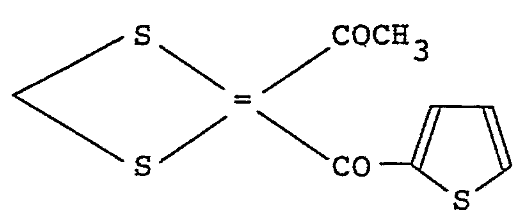

To a mixture of 1.68 g of 2-thiophencarbonylacetone 0.8 g of carbon disulfide, and 10 ml of dimethyl sulfoxide, well stirred at room temperature, was added 1.5

g of anhydrous potassium carbonate in small portions. After being stirred as it was for further 2 hours, the reaction liquid was cooled with ice water, and 2.68 g of diiodemethane was added thereto to effect reaction. After 3 hours of stirring at room temperature, water was added to the reaction mixture, and the resulting mixture was extracted with ethyl acetate. The ethyl acetate layer obtained was washed with water, dried over anhydrous sodium sulfate, and stripped of the solvent by evaporation to give crude crystals. The crystals were recrystallized from a solvent mixture consisting of isopropanol and n-hexane. Yield, 1.63 g (63.8% yield); m.p., 120°C.

Synthesis Example 5

Synthesis of 1-(1,3-dithietan-2-ylidene)-1-(4-fluorobenzoyl)acetophenone (compound No. 31)

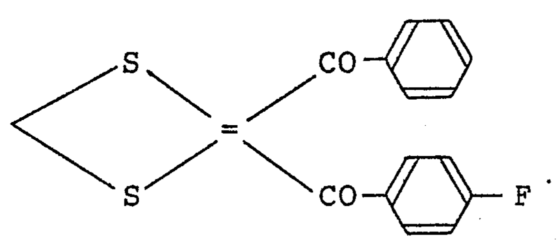

To a mixture of 2.42 g of benzoyl(p-fluorobenzoyl)methane 0.8 g of carbon disulfide, and 10 ml of dimethyl sulfoxide, well stirred at room temperature, was added 1.36 g of sodium ethylate. After being stirred as it was for further one hour, the reaction liquid was cooled with ice water, and 1.74 g of dibromomethane was added thereto to effect reaction. After 2 hours of

stirring at room temperature, water was added to the reaction mixture, and the resulting mixture was extracted three times with ethyl acetate. The ethyl acetate layer obtained was washed with water, dried over anhydrous sodium sulfate, and stripped of the solvent by evaporation to give crude crystals. The crystals were then purified by silica gel chromatography to obtain desired compound. Yield, 1.38 g (41.8% yield); m.p., 167° - 168°C.

Now, Examples regarding pharmaceutical compositions according to this invention will be described below. In the Examples, "part" is all part by weight. It is needless to say that the kinds and the proportions of the compounding ingredients used in the composition according to this invention can be changed variously without being restricted by these Examples.

Example 1

Compound No. 1 .................. 10 parts

Heavy magnesium oxide ........ 10 parts

Lactose ...................... 80 parts

The above ingredients were mixed uniformly and made into a medicinal preparation in the form of powders or fine granules.

Example 2

| | | |
|---|---|---|
| Compound No. 5 | ................ | 10 parts |
| Synthetic aluminum silicate | .. | 10 parts |
| Calcium hydrogen phosphate | ... | 5 parts |
| Lactose | ...................... | 75 parts |

The above ingredients were used to be made up into powders in a similar manner to that in Example 1.

Example 3

| | | |
|---|---|---|
| Compound No. 8 | ............... | 50 parts |
| Starch | ....................... | 10 parts |
| Lactose | ...................... | 15 parts |
| Crystalline cellulose | ........ | 20 parts |
| Polyvinyl alcohol | ............ | 5 parts |
| Water | ........................ | 30 parts |

The above ingredients were uniformly mixed, kneaded, then crushed, granulated, dried and sieved to obtain granules.

Example 4

A mixture of 99 parts of the granules obtained in Example 3 and 1 part of calcium stearate was compression-formed into tablets of 10 mm diameter.

Example 5

| | | |
|---|---|---|
| Compound No. 15 | ............... | 95 parts |
| Polyvinyl alcohol | ............ | 5 parts |
| Water | ........................ | 30 parts |

The above ingredients were made up into granules in the same manner as in Example 3. Ten parts of crystalline cellulose was added to 90 parts of the granules obtained above, and the mixture was compression-molded to obtain tablets of 8 mm diameter. The tablets may be further made up into dragée by using, in appropriate amounts, a mixed suspension of syrup gelatin and precipitated calcium carbonate, and a coloring agent.

Example 6

| | | |
|---|---|---|
| Compound No. 26 | ............... | 0.5 part |
| Nonionic surfactant | .......... | 2.5 parts |
| Physiological saline | ......... | 97 parts |

The above ingredients were mixed with warming, and then sterilized to obtain injections.

Example 7

The powders obtained in Example 1 were filled into capsule containers available on the market to obtain capsules.

Test Example 1

Effect of suppressing hepatic disorder caused by carbon tetrachloride

Test Method

The test compound was dissolved or suspended in olive oil, and orally administered at a dose of 250 mg/kg to mice (6 weeks of age, dd-strain, ♂). Six hours thereafter, carbon tetrachloride was orally administered in a proportion of 0.05 ml/kg. The animals were sacrificed 24 hours after the administration of carbon tetrachloride, and the extent of liver disorder was examined by visual observation.

On the other hand, blood was collected from the animal at the time of the sacrifice, and centrifuged to obtain plasma. The plasma glutamic piruvic transaminase (GPT) activity was determined according to the method of Reitman-Frankel. The activity was expressed in terms of Karmen Units (K.U.). The conditions of the liver were expressed in terms of liver disorder index as follows.

| Liver disorder index | Condition of liver |
|---|---|
| 0 | Healthyl liver |
| 2 | Slightly affected |
| 4 | Evidently observed disorder |
| 6 | Serious disorder |

Mice were used in groups of five and the results of test were represented by the mean value. When the GPT activity was 2,100 units or higher, or further determination was made, and the activity was calculated as 2,100 units for reasons of convenience.

The results obtained are shown in Table 2.

Table 2 Effect of carbon tetrachloride on liver disorder

| No. of compound of this invention | Liver disorder index | p-GPT (K.U.) |
|---|---|---|
| Administration of carbon tetrachloride alone | 5.4 | >2,100 |
| No treatment | 0 | 23 |
| 1 | 0.7 | 23 |
| 2 | 0.8 | 22 |
| 3 | 1.4 | 14 |
| 4 | 0.6 | 23 |
| 5 | 1.0 | 12 |
| 6 | 2.0 | 16 |
| 7 | 1.2 | 19 |
| 8 | 0.6 | 54 |
| 9 | 0.6 | 11 |
| 10 | 1.8 | 413 |
| 11 | 1.6 | 884 |
| 12 | 2.8 | 1739 |
| 13 | 0.8 | 13 |
| 14 | 0.2 | 73 |
| 15 | 0.5 | 203 |
| 16 | 0 | 15.8 |
| 17 | 0.4 | 15 |
| 18 | 1.6 | 1142 |
| 19 | 0.2 | 16 |
| 20 | 0 | 22 |

- cont'd -

Table 2 (cont'd)

| No. of compound of this invention | Liver disorder index | p-GPT (K.U.) |
|---|---|---|
| 21 | 0.2 | 14 |
| 22 | 0.5 | 71 |
| 23 | 6.6 | 1668 |
| 24 | 3.8 | 1654 |
| 25 | 1.2 | 205 |
| 26 | 0.9 | 36 |
| 27 | 3.2 | 210 |
| 28 | 1.0 | 42 |
| 29 | 3.4 | 233 |
| 30 | 0.6 | 19 |
| 31 | 0.4 | 16 |
| 32 | 0.8 | 18 |

WHAT IS CLAIMED IS:

1. A 1,3-diketone derivative represented by the general formula (I)

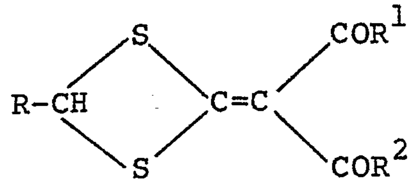

(I)

wherein $R^1$ denotes $C_1$-$C_5$ alkyl group; a phenyl group or a phenyl group substituted with halogen atom, $C_1$-$C_5$ alkyl group, $C_1$-$C_5$ alkoxy group; $R^2$ denotes $C_1$-$C_5$ alkyl group; a phenyl group; a phenyl group substituted with 1 to 3 groups selected from the group consisting of a halogen atom, $C_1$-$C_5$ alkyl group, $C_1$-$C_5$ alkoxy group, a phenyl group, a phenoxy group, and a benzyloxy group optionally having a halogen atom as a substituent on the phenyl ring; naphthyl group; a furyl group; a thienyl group; or a pyridyl group; provided that when R is hydrogen and $R^2$ is a methyl group or a phenyl group $R^1$ is a group other than a methyl or phenyl group; and R denotes a hydrogen atom, a methyl group or a phenyl group.

2. A 1,3-diketone derivative represented by the general formula (Ia)

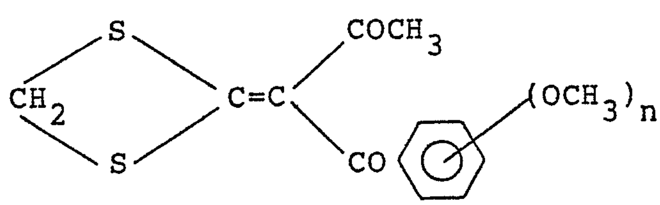

(Ia)

wherein n denotes an integer of 1 or 2.

3. A 1,3-diketone derivative according to Claim 1 or 2 which is 1-(2,4-dimethoxybenzoyl)-1-(1,3-dithietan-2-ylidene)acetone.

4. A 1,3-diketone derivative according to Claim 1 or 2 which is 1-(3,4-dimethoxybenzoyl)-1-(1,3-dithietan-2-ylidene)acetone.

5. A 1,3-diketone derivative according to Claim 1 or 2 which is 1-(1,3-dithietan-2-ylidene)-1-(2-methoxybenzoyl)acetone.

6. A 1,3-diketone derivative according to Claim 1 or 2 which is 1-(1,3-dithietan-2-ylidene)-1-(3-methoxybenzoyl)acetone.

7. A 1,3-diketone derivative according to Claim 1 or 2 which is 1-(1,3-dithietan-2-ylidene)-1-(4-methoxybenzoyl)acetone.

8. A pharmaceutical composition for treating hepatic disorders which comprises a pharmaceutically effective amount of a 1,3-diketone derivative represented by the general formula (I)

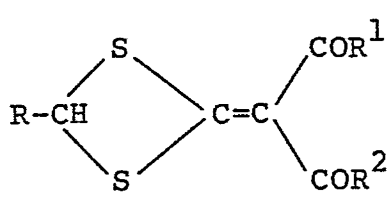

(I)

wherein $R^1$ denotes $C_1$-$C_5$ alkyl group; a phenyl group or a phenyl group substituted with halogen atom, $C_1$-$C_5$ alkyl

group, $C_1$-$C_5$ alkoxy group; $R^2$ denotes $C_1$-$C_5$ alkyl group; a phenyl group; a phenyl group substituted with 1 to 3 groups selected from the group consisting of a halogen atom, $C_1$-$C_5$ alkyl group, $C_1$-$C_5$ alkoxy group, a phenyl group, a phenoxy group, and a benzyloxy group optionally having a halogen atom as a substituent on the phenyl ring; naphthyl group; a furyl group; a thienyl group or a pyridyl group; R denotes a hydrogen atom a methyl group or a phenyl group as an active ingredient, and a pharmaceutically acceptable carrier.

9. A pharmaceutical composition for treating hepatic disorders according to Claim 8 wherein the 1,3-diketone derivative represented by the general formula (I) is a compound represented by the formula (Ia)

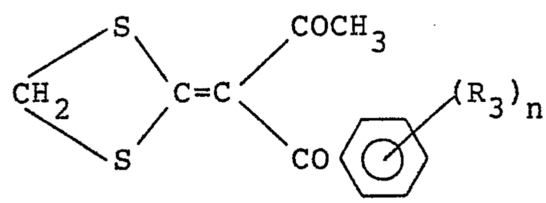

(Ia)

wherein $R^3$ denotes hydrogen or methoxy group, n denotes an integer of 1 or 2.

10. A pharmaceutical composition according to Claim 8 or 9 wherein the 1,3-diketone derivative is 1-(2,4-dimethoxybenzoyl)-1-(1,3-dithietan-2-ylidene)acetone.

11. A pharmaceutical composition according to Claim 8 or 9 wherein the 1,3-diketone derivative is 1-(3,4-dimethoxybenzoyl)-1-(1,3-dithietan-2-ylidene)-acetone.

12. A pharmaceutical composition according to Claim 8 or 9 wherein the 1,3-diketone derivative is 1-benzoyl-1-(1,3-dithietan-2-ylidene)acetone.

13. A pharmaceutical composition according to Claim 8 or 9 wherein the 1,3-diketone derivative is 1-(1,3-dithietan-2-ylidene)-1-(2-methoxybenzoyl)acetone.

14. A pharmaceutical composition according to Claim 8 or 9 wherein the 1,3-diketone derivative is 1-(1,3-dithietan-2-ylidene)-1-(3-methoxybenzoyl)acetone.

15. A pharmaceutical composition according to Claim 8 or 9 wherein the 1,3-diketone derivative is 1-(1,3-dithietan-2-ylidene)-1-(4-methoxybenzoyl)acetone.

16. A pharmaceutical composition according to Claim 8, wherein the said hepatic disorder is hepatitis.

17. A pharmaceutical composition according to Claim 8, wherein the said hepatic disorder is fatty liver.

18. A pharmaceutical composition according to Claim 8, wherein the said hepatic disorder is cholestasis.

19. A pharmaceutical composition according to Claim 8, wherein the compound is formulated into an administration unit form.

20. A pharmaceutical composition according to Claim 19, wherein the administration unit form is any one of powder, granule, tablet, pill, dragée, capsule, ampoule, suppository, suspension, liquid, emulsion, injection or instillation.

21. A pharmaceutical composition according to

Claim 19, wherein the administration unit form is injection or instillation.